(19) Europäisches Patentamt
European Patent Office
Office européen des brevets

(11) Veröffentlichungsnummer: **0 170 872**
**B1**

(12) **EUROPÄISCHE PATENTSCHRIFT**

(45) Veröffentlichungstag der Patentschrift: **23.08.89**

(51) Int. Cl.⁴: **C 07 C 103/42**

(21) Anmeldenummer: **85108116.6**

(22) Anmeldetag: **29.06.85**

(54) **Verfahren zur Herstellung, von 3,7-Bisacetaminoheptanon-2.**

(30) Priorität: **25.07.84 DE 3427400**
**13.07.84 DE 3425814**

(43) Veröffentlichungstag der Anmeldung:
**12.02.86 Patentblatt 86/7**

(45) Bekanntmachung des Hinweises auf die Patenterteilung:
**23.08.89 Patentblatt 89/34**

(84) Benannte Vertragsstaaten:
**AT BE CH DE FR GB IT LI NL**

(56) Entgegenhaltungen:
**DE-A-2 800 311**
**FR-A-2 375 219**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Scholl, Hans- Joachim, Dr., Rudolf-Sohmstrasse 28, D-5000 Koeln 60 (DE)**

**EP 0 170 872 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die vorliegende Erfindung betrifft ein Verfahren zur Herstellung von 3,7-Bisacetaminoheptanon-2, aus Lysin. Die Herstellung von 3,7-Bisbenzaminoheptanon-2 aus Lysin gemäß FR-A-2 375 219 Beispiel 1 (1) und (2) erfolgt in 2 Stufen. Demgegenüber ist durch das beanspruchten Verfahren das 3,7-Bisacetaminoheptanon-2 in einem Eintopfverfahren in guten Ausbeuten zugänglich.

Das Verfahren zur Herstellung von 3,7-Bisacetaminoheptanon-2, der Formel (I)

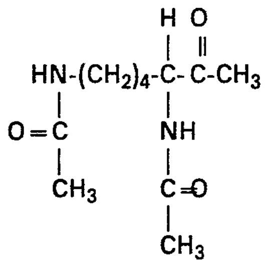

(I)

ist dadurch gekennzeichnet, daß man L-Lysin in freier Form, als Racemat, in seiner D-Form, vorzugsweise in seiner L-Form, in freier Form und/oder in seiner HCl-Salz-Form mit Essigsäureanhydrid in Gegenwart einer tertiären organischen Aminbase und unter Zusatz eines 4-Aminopyridin-Derivates umsetzt.

Bezogen auf 1 Mol Lysin und/oder Lysin-hydrochlorid können beispielsweise 4 bis 10 Mol Essigsäureanhydrid eingesetzt werden. Vorzugsweise beträgt diese Menge 6 bis 8 Mol.

Als tertiäre organische Aminbasen kommen beispielsweise Trialkylamine in Frage, die Alkylgruppen mit 2 bis 6 C-Atomen enthalten. Bevorzugt wird Triethylamin eingesetzt. Bezogen auf 1 Mol Lysin und/oder Lysin-hydrochlorid können beispielsweise 3 bis 8 Mol einer tertiären organischen Aminbase eingesetzt werden. Vorzugsweise beträgt diese Menge 4 bis 6 Mol.

Als 4-Aminopyridin-Derivate kommen beispielsweise solche der Formel (II) in Frage

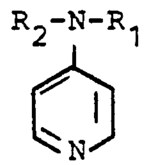

(II),

in der

$R_1$ und $R_2$ unabhängig voneinander für einen einbindigen $C_1$- bis $C_6$-Alkylrest oder $R_1$ und $R_2$ gemeinsam für einen zweibindigen $C_3$- bis $C_5$-Alkylrest stehen.

Bevorzugt wird N,N-Dimethyl-4-aminopyridin oder 4-Pyrrolidino-pyridin eingesetzt. Bezogen auf Lysin und/oder L-Lysin-hydrochlorid können beispielsweise 0,1 bis 5 Gew.-% eines 4-Aminopyridin-Derivates eingesetzt werden. Vorzugsweise beträgt diese Menge 0,5 bis 3 Gew.-%.

Eine beispielhafte Ausführungsform des erfindungsgemäßen Verfahrens wird durch folgendes Formelschema erläutert:

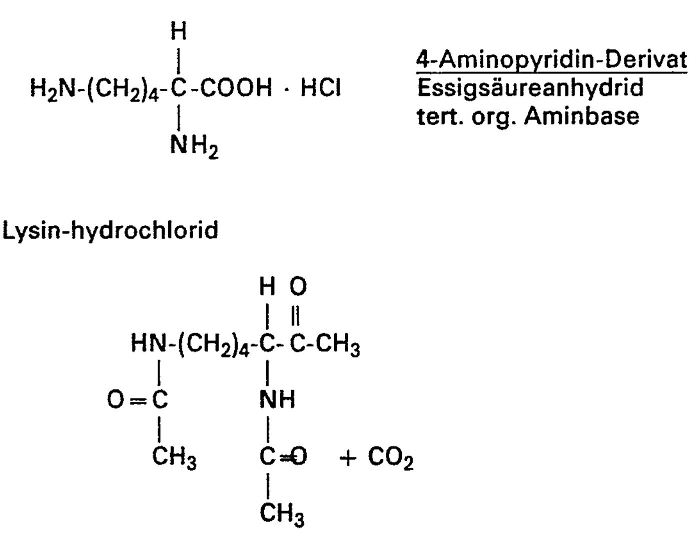


Lysin-hydrochlorid

(I)

Der Ablauf der Reaktion kann über die entwickelte Menge $CO_2$ verfolgt werden. Unterbleibt z. B. der Zusatz eines 4-Aminopyridin-Derivates, so wird keine $CO_2$-Entwicklung beobachtet. Die Reaktion wird im allgemeinen solange durchgeführt, bis die $CO_2$-Entwicklung beendet ist.

Geeignete Reaktionstemperaturen sind beispielsweise solche im Bereich von 20 bis 100°C. Vorzugsweise liegt die Reaktionstemperatur im Bereich von 30 bis 70°C.

Der Zusatz eines Lösungsmittels ist im allgemeinen nicht erforderlich, da das Essigsäureanhydrid und die tertiäre organische Aminbase gleichzeitig als Lösungsmittel dienen können.

Die Aufarbeitung des nach Beendigung der Reaktion vorliegenden Reaktionsgemisches kann beispielsweise so erfolgen, daß man, falls das Hydrochlorid der eingesetzten tertiären organischen Aminbase ausgefallen ist, dieses abfiltriert, aus dem Filtrat oder, falls keine festen Bestandteile vorliegen, aus dem gesamten Reaktionsgemisch niedrig siedende Anteile im Vakuum entfernt, das verbleibende Rohprodukt in ein geeignetes Lösungsmittel einrührt, z. B. in Essigsäureethylester oder Toluol und den entstehenden Niederschlag abfiltriert, wäscht und trocknet.

Man erhält so 3,7-Bisacetaminoheptanon-2 in guten Ausbeuten und Reinheiten ohne merkliche Nebenreaktionen. Dieses Ergebnis ist ausgesprochen überraschend, da zum einen bei einer derart komplexen Reaktion ein eindeutiger Reaktionsverlauf nicht zu erwarten war und zum anderen ein trifunktionelles Ausgangsprodukt eingesetzt wird (Lysin), das im Prinzip zu einer Fülle von Reaktionen befähigt ist.

Es wurde weiterhin gefunden, daß man 3,7-Bisacetaminoheptanon-2 zur Herstellung von Salzen des 3,7-Bisaminoheptanons-2 verwenden kann, indem man es einer sauren Hydrolyse unterwirft.

Diese Reaktion kann beispielsweise durch folgendes Formelschema erläutert werden:

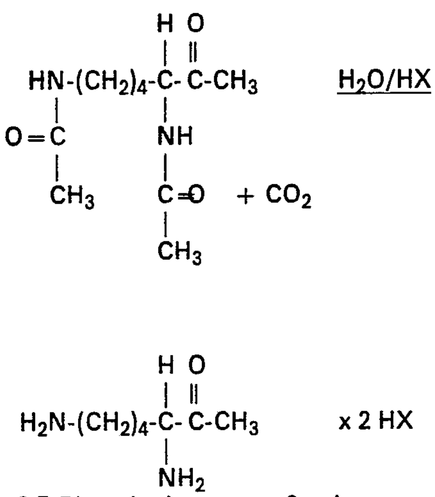


3,7-Bisaminoheptanon-2-salz X = Säurerest

Diese Hydrolyse kann beispielsweise mit überschüssigen, wäßrigen, organischen oder anorganischen Säuren durchgeführt werden durch mehrstündiges Kochen am Rückfluß. Als Säuren kommen insbesondere starke Säuren in Frage, wie Toluolsulfonsäure, Salzsäure oder Schwefelsäure. Das Hydrolysegemisch kann man beispielsweise aufarbeiten, indem man zunächst im Vakuum flüchtige Bestandteile abtrennt, daß Rohprodukt mit einem geeigneten Lösungsmittel verrührt, z. B. Aceton, abfiltriert und trocknet.

Salze des 3,7-Bisaminoheptanon-2 sind bekannte und wertvolle Zwischenprodukte. Gemäß der DE-A-2 755 749 die der FR-A-2 375 219 äquivalent ist, können daraus durch Umsetzung mit Thiocyanaten Imidazol-2-thione hergestellt werden, die ihrerseits Vorprodukte für Verbindungen mit pharmakologischer Wirksamkeit gegenüber Histamin-$H_2$-Rezeptoren sind oder $H_2$-Antagonisten (siehe DE-A-2 755 749).

Die in der DE-A-2 755 749 beschriebene Herstellung von 3,7-Bisaminoheptanon-2-salzen ist ein mehrstufiges Verfahren, das für eine Anwendung im technischen Maßstab nicht geeignet ist. Über das neue 3,7-Bisacetaminoheptanon-2 sind Salze des 3,7-Bisaminoheptanons-2 in beliebiger Menge auf einfache Weise zugänglich.

Das folgende Beispiel erläutert die Erfindung.

**Beispiel**

In eine Mischung aus 1230 g Essigsäureanhydrid und 810 g Triethylamin wurden 365 g L-Lysinhydrochlorid und 5 g 4-Pyrrolidino-pyridin unter Rühren eingetragen.

Die Reaktionstemperatur wurde bei 40 bis 50°C bis zur Beendigung der Gasentwicklung (ca. 40 l) gehalten. Dann wurde das ausgefallene Triethylaminohydrochlorid abfiltriert und das Filtrat im Vakuum von niedrigsiedenden Anteilen befreit.

Das verbleibende Rohprodukt wurde in 3 Liter Essigester eingerührt, der entstehende Niederschlag abgesaugt, mit Essigester nachgewaschen und getrocknet. Es wurden 402 g (88 % der Theorie) 3,7-

Bisacetamino-heptanon-2 (II) mit einem Schmelzpunkt von 111 bis 113°C erhalten.

Analyse:

| (II) | % C | % H | % N |
|---|---|---|---|
| gefunden: | 57,7 | 8,7 | 12,2 |
| Theorie: | 57,9 | 8,8 | 12,3 |

Kernresonanzspektrum:

$^1$H-NMR (60 MHz, CD, $Cl_3$)δ:
NH (breit 6,4 und 6,8), CH (breit 4,5), $CH_2$ (breit, zentriert bei 3,2), $CH_3$ (Singuletts bei 2,0, 2,06, 2,23), $-(CH_2)_3$ (Multiplett, zentriert bei 1,5).

**Patentansprüche**

1. Verfahren zur Herstellung von 3,7-Bisacetaminoheptanon-2, dadurch gekennzeichnet, daß man Lysin in freier Form und/oder in seiner HCl-Salz-Form mit Essigsäureanhydrid in Gegenwart einer tertiären organischen Aminbase und unter Zusatz eines 4-Aminopyridin-Derivates umsetzt.
2. Verfahren nach Anspruch 1, dadurch gekennzeichnet, daß man bezogen auf 1 Mol Lysin und/oder Lysin-hydrochlorid 4 bis 10 Mol Essigsäureanhydrid einsetzt.
3. Verfahren nach Ansprüchen 1 oder 2, dadurch gekennzeichnet, daß man bezogen auf 1 Mol Lysin und/oder Lysin-hydrochlorid 3 bis 8 Mol einer tertiären organischen Aminbase einsetzt.
4. Verfahren nach Ansprüchen 1 bis 3, dadurch gekennzeichnet, daß man als tertiäre organische Aminbasen ein Trialkylamin einsetzt, das Alkylgruppen mit 2 bis 6 C-Atomen enthält.
5. Verfahren nach Ansprüchen 1 bis 4, dadurch gekennzeichnet, daß man bezogen auf Lysin und/oder Lysinhydrochlorid 0,1 bis 5 Gew.-% eines 4-Aminopyridin-Derivates einsetzt.
6. Verfahren nach Ansprüchen 1 bis 5, dadurch gekennzeichnet, daß man ein 4-Aminopyridin-Derivat der Formel

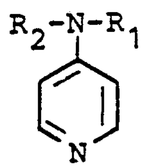

einsetzt, in der
$R_1$ und $R_2$ unabhängig voneinander für einen einbindigen $C_1$- bis $C_6$-Alkylrest oder $R_1$ und $R_2$ gemeinsam für einen zweibindigen $C_3$- bis $C_5$-Alkylrest stehen.

**Claims**

1. A process for the production of 3,7-bisacetamino-2-heptanone, characterized in that lysine in free form and/or in the form of its HCl salt is reacted with acetic anhydride in the presence of a tertiary organic amine base and with addition of a 4-aminopyridine derivative.
2. A process as claimed in claim 1, characterized in that 4 to 10 mol acetic anhydride are used per mol lysine and/or lysine hydrochloride.
3. A process as claimed in claim 1 or 2, characterized in that 3 to 8 mol of a tertiary organic amine base are used per mol lysine and/or lysine hydrochloride.
4. A process as claimed in claims 1 to 3, characterized in that a trialkylamine containing $C_2$-$C_6$ alkyl groups is used as the tertiary organic amine base.
5. A process as claimed in claims 1 to 4, characterized in that 0.1 to 5 % by weight of a 4-aminopyridine derivative, based on lysine and/or lysine hydrochloride, is used.
6. A process as claimed in claims 1 to 5, characterized in that a 4-aminopyridine derivative corresponding to the formula

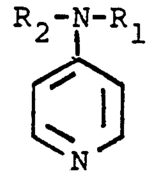

in which
$R_1$ and $R_2$ independently of one another represent a $C_1$-$C_6$ alkyl radical with one bond or $R_1$ and $R_2$ together represent a $C_3$-$C_5$ alkyl radical with two bonds, is used.

## Revendications

1. Procédé de préparation de 3,7-bisacétaminoheptanone-2, caractérisé en ce qu'on fait réagir de la lysine sous forme libre et/ou sous forme de son sel de HCl avec de l'anhydride d'acide acétique en présence d'une base d'amine tertiaire organique et avec addition d'un dérivé de 4-aminopyridine.

2. Procédé selon la revendication 1, caractérisé en ce que, rapporté à 1 mole de lysine et/ou de chlorhydrate de lysine, on utilise 4 à 10 moles d'anhydride d'acide acétique.

3. Procédé selon les revendications 1 ou 2, caractérisé en ce que, rapporté à 1 mole de lysine et/ou de chlorhydrate de lysine, on utilise 3 à 8 moles d'une base d'amine tertiaire organique.

4. Procédé selon les revendications 1 à 3, caractérisé en ce que, comme bases d'amines tertiaires organiques, on utilise une trialkylamine contenant des groupes alkyle comportant 2 à 6 atomes de carbone.

5. Procédé selon les revendications 1 à 4, caractérisé en ce que, rapporté à la lysine et/ou au chlorhydrate de lysine, on utilise 0,1 à 5 % en poids d'un dérivé de 4-aminopyridine.

6. Procédé selon les revendications 1 à 5, caractérisé en ce qu'on utilise un dérivé de 4-aminopyridine de formule:

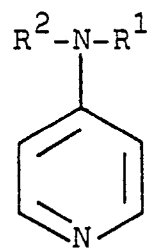

dans laquelle
$R^1$ et $R^2$ représentent indépendamment l'un de l'autre un radical alkyle en $C_1$-$C_6$ à une liaison ou $R^1$ et $R^2$ ensemble représentent un radical alkyle en $C_3$-$C_5$ à deux liaisons.